# EUROPEAN PATENT APPLICATION

(11) **EP 2 438 933 A1**
(43) Date of publication of application: **11.04.2012**
(21) Application number: 10075485.2
(22) Date of filing: 17.09.2010
(51) Int. Cl.: A61L 29/08, A61L 29/16

(54) **Device for use in treatment of heart valve disease and endocarditis**

(71) Applicant: Eurocor Gmbh, 53227 Bonn (DE)
(72) Inventor: von Strandmann, R. Pogge, 50933 Köln (DE)
(74) Representative: Arth, Hans-Lothar

(57) **Abstract**

The present invention relates to a method for coating catheter balloons, preferably textured catheter balloons and preferably valvuloplasty catheter balloons, with a therapeutic amount of an anti-infective agent and optionally a biological and biodegradable carrier substance and optionally further components. Moreover, the present invention relates to catheter balloons and preferably valvuloplasty catheter balloons coated with a therapeutic amount of an anti-infective agent and optionally a carrier substance obtainable according to the coating methods disclosed herein as well as the use of such coated catheter balloons for treatment of heart valve stenosis and release of the anti-infective agent or agents for prophylaxis of endocarditis especially endocarditis caused as a result of valvuloplasty. The coated catheter balloons can be used alone or in combination with a coated or uncoated valve replacement device on the catheter balloon before or after the coating with the therapeutically anti-infective agent and optionally carrier substance.

## Description

The present invention relates to a method for coating catheter balloons, preferably textured catheter balloons and preferably valvuloplasty catheter balloons, with a therapeutic amount of an anti-infective agent and optionally a biological and biodegradable carrier substance and optionally further components. Moreover, the present invention relates to catheter balloons and preferably valvuloplasty catheter balloons coated with a therapeutic amount of an anti-infective agent and optionally a carrier substance obtainable according to the coating methods disclosed herein as well as the use of such coated catheter balloons for treatment of heart valve stenosis and release of the anti-infective agent or agents for prophylaxis of endocarditis especially endocarditis caused as a result of valvuloplasty. The coated catheter balloons can be used alone or in combination with a coated or uncoated valve replacement device on the catheter balloon before or after the coating with the therapeutically anti-infective agent and optionally carrier substance.

The heart is a myogenic muscular organ found in all animals with a circulatory system including vertebrates. It is the most heavily worked muscle in the body contracting about three billion times during the course of a human lifetime as the heart is responsible for pumping blood throughout the blood vessels by repeated, rhythmic contractions. It consists of four chambers, two upper atria and two lower ventricles. The heart has four valves: the tricuspid, pulmonary, mitral, and aortic valves which are located between the atria and ventricles. The valves have tissue flaps that open and close with each heartbeat and maintain coordinated unidirectional blood flow from the upper atria to the lower ventricles.

The aortic valve lies within the aorta at its origin from the left ventricle and prevents reflux of blood from the aorta into the left ventricle. Aortic valves can be affected by several processes. In aortic insufficiency, also called aortic regurgitation, the aortic valve is incompetent and blood flows passively back to the heart in the wrong direction. In contrast, the valve fails to open fully in aortic stenosis, thereby obstructing blood flow out from the heart. Symptoms of aortic stenosis include progressive shortness of breath on exertion (exertional dyspnoea). This dyspnoea may be so subtle that affected persons are not consciously aware of but may inadvertently cut down on exertional activities. More severe symptoms include syncope, chest pain and angina pectoris that could lead to heart failure, endocarditis and/or sudden cardiac death.

Aortic valve stenosis is the most significant valvular disease in the Western world. Its incidence is expected to grow with the continuing increase in longevity. Severe aortic valve stenosis is associated with severe morbidity. Affected persons' death may occur within two to three years of symptom onset. Aortic valve replacement is the definite therapy for severe aortic stenosis [Dvir D, Assali A, Vaknin H, Sagie A, Shapira Y, Battler A, Porat E, Kornowski R. Percutaneous aortic valve implantation: early clinical experience and future perspectives. Isr Med Assoc J. 2009;11:244-9; Vahanian A, Baumgartner H, Bax J, Butchart E, Dion R, Filippatos G, Flachskampf F, Hall R, lung B, Kasprzak J, Nataf P, Tornos P, Torracca L, Wenink A; Task Force on the Management of Valvular Hearth Disease of the European Society of Cardiology; ESC Committee for Practice Guidelines. Guidelines on the management of valvular heart disease: The Task Force on the Management of Valvular Heart Disease of the European Society of Cardi-ology. Eur Heart J. 2007;28:230-68]. A balloon valvuloplasty of the stenotic aortic valve is always performed before the prosthetic valve is implanted. A balloon catheter is placed either transarterially or transapically by way of the delivery catheter and then expanded under tachycardic ventricular stimulation [Bleiziffer S, Ruge H, Mazzitelli D, Schreiber C, Hutter A, Krane M, Bau-ernschmitt R, Lange R. Valve implantation on the beating heart: catheter-assisted surgery for aortic stenosis. Dtsch Arztebl Int. 2009;106:235-41]. However, there are several limitations in the octogenarian and nonagenarian populations [Hara H, Pedersen WR, Ladich E, Mooney M, Virmani R, Nakamura M, Feld-man T, Schwartz RS. Percutaneous balloon aortic valvuloplasty revisited: time for a renaissance? Circulation. 2007;115:e334-8].

Balloon valvuloplasty as the sole treatment plays a very limited role in adults because its efficacy is low and complication rate is high (> 10%). Restenosis and clinical deterioration occur within 6 to 12 months in most patients resulting in a mid-term and long-term outcome similar to natural history [Connolly HM, Oh JK, Schaff HV, Roger VL, Osborn SL, Hodge DO, Tajik AJ. Severe aortic stenosis with low transvalvular gradient and severe left ventricu-lar dysfunction: result of aortic valve replacement in 52 patients. Circulation. 2000;101:1940-6]. The problem of restenosis in aortic valve stenosis was recently addressed and solved by European patent application EP08750817.2 the entire contents of which are incorporated herein by reference.

Usually, balloon valvuloplasty is performed on the patient on the first day after admission, followed by external beam radiation therapy the next day for three consecutive days, and, finally, discharge of the patient on the fifth day, successful treatment provided. The invention of the European patent application EP08750817.2 reduces the number of hospitalisation days, consumption of material and input of human resources. Last but not least, quality of life will improve considerably for the patients if hospitalisation is as short as possible.

Furthermore, balloon valvuloplasty can be considered as a bridge to surgery in haemodynamically unstable patients who are at high risk for surgery. Patients are at high risk for surgery if they are 1) asymptomatic and were classified by severe aortic stenosis and abnormal exercise test showing ventricular arrhythmias, or 2) asymptomatic with excessive left ventricular hypertrophy ≥: 15 millimetre unless this is due to hypertension, or 3) patients showing symptoms of aortic stenosis with low gradient (< 40 mmHg) and left ventricular dysfunction without contractile reserve. Also, patients with symptomatic severe aortic stenosis who require urgent major non-cardiac surgery may be eligible for balloon valvuloplasty. Occasionally balloon valvuloplasty could be considered as a palliative measure in individual cases when surgery is contraindicated because of severe comorbidity. Moreover, balloon angioplasty plays an important role in the paediatric population. Also, in patients with severe aortic stenosis who remain symptomatic despite diuretics, balloon aortic angioplasty can be considered during pregnancy but experience is limited with this procedure during pregnancy [Vahanian A, Baumgartner H, Bax J, Butchart E, Dion R, Filippatos G, Flachskampf F, Hall R, lung B, Kasprzak J, Nataf P, Tornos P, Torracca L, Wenink A; Task Force on the Management of Valvular Hearth Disease of the European Society of Cardiology; ESC Committee for Practice Guidelines. Guidelines on the management of valvular heart disease: The Task Force on the Management of Valvular Heart Disease of the European Society of Cardi-ology. Eur Heart J. 2007;28:230-68].

The highest risk of balloon valvuloplasty lies in the development of aortic insufficiency due to the aortic valve not being able to seal the aorta from the left ventricle. Aortic valve stenosis also increases the risk of bacteria entering the bloodstream and causing an infection in of the stenosed valve called infective endocarditis. Narrowed aortic valves are more prone to infection than healthy ones. Because of the higher risk of infection of the damaged aortic valves by bacteria, affected persons have to be treated with antibiotics for prevention of endocarditis before certain medical procedures such as dental work or major operations. Consequently, balloon angioplasty is contraindicated in patients with infective endocarditis.

Infective endocarditis is an endovascular infection usually caused by bacteria that affects not only the native heart valves but intravascular implanted foreign material such as pacemaker electrodes or valvular prostheses with increasing frequencies. Infective endocarditis is a peculiar disease as neither incidence nor mortality of the disease has decreased in the past 30 years. Although major advances in both diagnostic and therapeutic procedures have been made infective endocarditis still carries a poor prognosis, high morbidity and a mortality of 20 to 30 percent.

Then, infective endocarditis is not a uniform disease, but presents in a variety of different forms. The epidemiological profile of infective endocarditis has changed substantially over the last few years [Task Force on the Prevention, Diagnosis, and Treatment of Infective Endocar-ditis of the European Society of Cardiology; European Society of Clinical Mi-crobiology and Infectious Diseases; International Society of Chemotherapy for Infection and Cancer, Habib G, Hoen B, Tornos P, Thuny F, Prendergast B, Vilacosta I, Moreillon P, de Jesus Antunes M, Thilen U, Lekakis J, Lengyel M, Müller L, Naber CK, Nihoyannopoulos P, Moritz A, Zamorano JL; ESC Com-mittee for Practice Guidelines, Vahanian A, Auricchio A, Bax J, Ceconi C, Dean V, Filippatos G, Funck-Brentano C, Hobbs R, Kearney P, McDonagh T, McGregor K, Popescu BA, Reiner Z, Sechtem U, Sirnes PA, Tendera M, Var-das P, Widimsky P. Guidelines on the prevention, diagnosis, and treatment of infective endocarditis (new version 2009): the Task Force on the Prevention, Diagnosis, and Treatment of Infective Endocarditis of the European Society of Cardiology (ESC). Eur Heart J. 2009;30:2369-413]. The incidence of infective endocarditis ranges from one country to another within 3-10 episodes/100,000 person-years reflecting methodological differences between the surveys. The incidence of infective endocarditis was very low in young patients but increased dramatically with age. Incidence reached its peak in patients between 70 and 80 years with 14.5 episodes/100,000 person-years.

Infective endocarditis should be regarded as a set of clinical situations which are sometimes very different from each other. Four categories of infective endocarditis must be separated: Infective endocarditis according to localization of infection and presence or absence of intracardiac material, infective endocarditis according to the mode of acquisition, active infective endocarditis, and recurrence.

According to the microbiological findings the following categories are proposed by [Habib G. et al.] as cited above. The most important category of the diseases, infective endocarditis with positive blood cultures, represents around 85 percent of all cases of infective endocarditis. Causative microorganisms are most often staphylococci, streptococci and enterococci.

The next category is infective endocarditis with negative blood cultures because of prior treatment with antibiotics. This applies to such cases where patients received antibiotics for unexplained fever before any blood cultures were performed and diagnosis of infective endocarditis had not been considered.

Also, infective endocarditis is frequently associated with negative blood cultures. Cases are usually due to fastidious organisms such as nutritionally variant streptococci, fastidious Gram-negative bacilli of the genera *Haemophilus, Actinobacillus, Cardiobacterium, Eikenalla* and *Kingella.* Further causative pathogens include *Brucella*, a Gram-negative bacterium causing brucellosis and fungi.

The last category is characterised by infective endocarditis associated with constantly negative blood cultures. These forms are caused by intracellular bacteria such as *Coxellia burnetii, Chlamydia* and *Bartonella* and account for up to five percent of all cases of infective endocarditis.

Normally, the valve endothelium is resistant to colonization and infection by circulating bacteria. Mechanical disruption of the endothelium, however, results in exposure of underlying extracellular matrix proteins plus production of tissue factor. Deposition of fibrin and platelets is part of the normal healing process. This is also known as non-bacterial thrombotic endocarditis (NBTE). Unfortunately, NBTE facilitates bacterial adherence and subsequent infection triggered by damage to the endothelium. Damage to the endothelium may result from mechanical lesion, provoked by catheters or electrodes for example, or degenerative changes in elderly individuals. In the absence of valve lesions endothelial inflammation may promote infective endocarditis, too. Local inflammation triggers endothelial cells to express adhesion molecules, e.g. integrin. If such activated endothelial cells bind fibronectin they provide an adhesive surface to circulating *Staphylococcus aureus* and other pathogens of infective endocarditis expressing fibronectin-binding proteins on their surface. Once adherent, *Staphylococcus aureus* trigger their active internalization into valve endothelial cells. One scenario is that bacteria persist and escape host defences and antibiotics. In a second scenario, they multiply and may spread to distant organs.

The principle of prophylaxis for infective endocarditis was developed on the basis of observational studies in the early 20th century. The basic hypothesis is based on the assumption that bacteraemia subsequent to medical procedures can cause infective endocarditis. Particularly, patients with predisposing factors are at risk. Prophylactic treatment with antibiotics can prevent infective endocarditis in these patients my minimizing or preventing bacteraemia. Also, alterations in bacterial properties leading to reduced bacterial adherence to the endothelial surface are possible. Patients being at highest risk of endocarditis are those who have artificial heart valves. Germs are more likely to attach to an artificial (prosthetic) heart valve than to a normal heart valve. The risk of infection is highest in the first year after implantation. Patients who have congenital heart defects may have hearts more susceptible to infection. Also, patients with a prior history of endocarditis are at highest risk as an episode of endocarditis damages heart tissue and valves, increasing the risk of a future heart infection. Then, certain medical conditions such as rheumatic fever or infection can damage or scar one or more of the heart valves, making them more prone to endocarditis. Finally, people who use illegal drugs by injecting them are at a greater risk of endocarditis. The needles used to inject drugs are often contaminated with the bacteria that can cause endocarditis.

Prior studies have reported significant mortality and morbidity in patients with bicuspid aortic valve related to the development of aortic valve dysfunction, endocarditis, and dissection [Tzemos N, Therrien J, Yip J, Thanassoulis G, Tremblay S, Jamorski MT, Webb GD, Siu SC. Outcomes in adults with bicuspid aortic valves. JAMA. 2008;300:1317-25]. The problem of restenosis in aortic valve stenosis was recently addressed and solved by European patent application EP08750817.2. At present, the long-standing problem of prevention of bacterial or fungal infection during and after balloon valvuloplasty has not been solved by the current state of the art. Therefore, adequate treatment of possible infection that could cause bacteraemia or fungaemia at the time of balloon valvuloplasty and effective management to prevent conditions that can lead to chronic or repeated infections are essential in reducing the risk of subsequent endocarditis. Objective of the present invention is application of at least one therapeutically anti-infective agent to the surface of a catheter balloon in such manner that the created coating is easily detachable from the balloon surface upon inflation of the catheter balloon and will be transferred efficiently to the tissue of the heart valves to prevent infective endocarditis. In the context of this application, the terms "anti-infective" and "anti-microbial" are used interchangeably and include the terms "anti-bacterial" and "anti-fungal".

Said objective is resolved by the technical teachings of the independent claims. Further advantageous embodiments of the invention result from the dependent claims, the description and the examples.

The inventors found surprisingly that a coated catheter balloon and a coating method as described herein is especially suited for resolving said objective of being easily detachable from the balloon surface upon inflation of the catheter balloon with subsequent efficient transfer to the valve tissue. Said method for loading or coating expandable catheter balloons comprises the following steps:
I) Providing an uncoated catheter balloon;
   and
IIA) Providing a solution of at least one therapeutically agent against endocarditis and optionally at least one carrier substance;
   or
IIB) Providing a solution of at least one therapeutically agent against endocarditis and optionally providing a solution of at least one carrier substance;
   and
IIIA) Coating the surface of the catheter balloon with the solution of at least one therapeutically agent against endocarditis and optionally at least one carrier substance;
   or
IIIB) Coating the surface of the catheter balloon with the solution of at least one therapeutically agent against endocarditis and subsequently with optionally a solution of at least one carrier substance or coating the surface of the catheter balloon with optionally a solution of at least one carrier substance and subsequently with the solution of at least one therapeutically agent against endocarditis;
IV) Drying the coated catheter balloon.

The catheter balloon of the inventive balloon catheter is preferably a catheter balloon of a valvuloplasty catheter.

The solution used for coating the surface of the balloon of a catheter contains at least one therapeutically anti-infective agent against endocarditis that is selected from the following group comprising or consisting of:
Ampicillin, bacampicillin, carbenicillin, indanyl, mezlocillin, piperacillin, ticarcillin, amoxicillin-clavulanic acid, ampicillin-sulbactam, benzylpenicillin, cloxacillin, diclox-acillin, methicillin, oxacillin, penicillin G, penicillin V, piperacillin, tazobactam, ticarcillin, clavulanic acid, nafcillin, cephalosporin of the first generation, cefadroxil, cefazolin, cephalexin, cephalothin, cephapirin, cephradine, cefaclor, cefamandol, cefonicid, cefotetan, cefoxitin, cefprozil, ceftmetazole, cefuroxime, loracarbef, cefdinir, ceftibuten, cefoperazone, cefixime, cefotaxime, cefpodoxime proxetil, ceftazidime, ceftizoxime, ceftriaxone, cefepime, azithromycin, clarithromycin, clindamycin, dirithromycin, erythromycin, roxithromycin, telithromycin, cethromycin, spiramycin, ansymacin, oelandomycin, lincomycin, troleandomycin, cinoxacin, ciprofloxacin, enoxacin, gatifloxacin, grepafloxacin, levofloxacin, lomefloxacin, moxifloxacin, nalidixic, acid, norfloxacin, ofloxacin, sparfloxacin, trovafloxacin, oxolinic, acid, gemifloxacin, perfloxacin, imipenem-cilastatin, meropenem, aztreonam, amikacin, gentamicin, kanamycin, neomycin, netilmicin, streptomycin, tobramycin, paromomycin, teicoplanin, vancomycin, demeclocycline, doxycycline, methacycline, minocycline, oxytetracycline, tetracycline, chlortetracycline, tigecycline, mafenide, silver sulfadiazine, sulfacetamide, sulfadiazine, sulfamethoxazole, sulfasalazine, sulfisoxazole, trimethoprim-sulfamethoxazole, sulfamethizole, rifabutin, rifampin, rifapentine, linezolid, streptogramins, quinopristin, dalfopristin, bacitracin, chloramphenicol, fosfomycin, isoniazid, methenamine, metronidazol, mupirocin, nitrofurantoin, nitrofurazone, novobiocin, polymyxin, spectinomycin, trimethoprim, colistin, cycloserine, capreomycin, ethionamide, pyrazinamide, para-aminosalicyclic acid, erythromycin-2-acetate, erythromycin-2-stearate, erythromycin estolate, erythromycin ethylsuccinate, erythromycin glutamate, erythromycin lactic propionate, rifampicin, miconazole, ketoconazole, clotrimazole, econazole, bifonazole, butoconazole, fenticonazole, isoconazole, oxiconazole, sertaconazole, sulconazole, tioconazole, fluconazole, itroconauzole, isavuconazole, ravuconazole, posaconazole, voriconazole, teraconazole, abfungin, terbinafine, amorolfine, naftifine, butenafine, anidulafungin, caspofungin, micafungin, benzoic acid, ciclopirox olamine, tolnaftate, undecyclenic acid, 5-fluorocytosine, griseofulvin, haloprogin, fusidic acid, gramicidin, pristinamycin, ramoplanin, tyrotricin, natamycin, rimocidin, filipin, nystatin, amphotericin B, candicin, hamycin, sirolimus (rapamycin), biolimus A9, everolimus, myolimus, novolimus, pimecrolimus, ridaforolimus, tacrolimus FK 506, temsirolimus, zotarolimus and/or mixtures of the aforementioned agents.

The therapeutically anti-infective agent against endocarditis has preferably lipophilic properties. Clinical benefit in treatment of so-called "difficult to treat infections" has been proven for said therapeutically anti-infective agent. The therapeutically anti-infective agent against endocarditis is preferably selected from the group comprising all compounds with anti-microbial properties such as lipophilic compounds selected from the group of amino glycosides, the group of cephalosporines and beta-lactames related thereon, chloramphenicol, lincosamide, macrolides, penicillins, chinolons, sulfonamides, fluoroquinolones, tetracyclines and/or mixtures of the aforementioned agents. In addition to these anti-infective agents the use of silver and more preferred of copper is possible. Release of small desired amounts of elemental silver particles or copper particles (such as mikro particles or nano particles or micelles containing the silver or copper) also exhibits an anti-infective effect and is useful for the prophylaxis and treatment of endocarditis.

Therapeutically anti-infective agents against endocarditis are most preferred lipophilic anti-infective agents selected from the group comprising: Benzathin, penicillin G, penicillin V, chloramphenicol, chlortetracycline (aureomycin), ciprofloxacin, clarithromycin, clindamycin-palmitathydrochloride, trimethoprim, erythromycin-2-acetate, erythromycin-2-stearate, erythromycin estolate, erythromycin ethylsuccinate, erythromycin glutamate, erythromycin lactic propionate, rifampicin, fusidic acid and preferably free fusidic acid, gramicidin, mupirocin, lipophilic agents from the group of imidazoles such as econazole, itraconazole, clotrimazole and others, pristinamycin, silver sulfadiazine, ciprofloxacin, enoxacin, gatifloxacin, grepafloxacin, levofloxacin, lomefloxacin, moxifloxacin, nalidixic, acid, norfloxacin, ofloxacin, sparfloxacin, trovafloxacin, oxolinic, acid, gemifloxacin, perfloxacin, teicoplanin, ramoplanin, pristinamycin due to its lipophilic alkyl group and tyrothricin due to its insolubility in water.

The at least one therapeutically anti-infective agent against endocarditis is applied to the surface of an expandable balloon of a balloon catheter according to the method disclosed above. Expandable balloons of any commercially available sic conventional balloon catheters deployed for valvuloplasty such as the Inoue balloon catheter can be used for the present invention. Preferably, balloons of balloon catheters suited for valvuloplasty as described in European patent application EP08750817.2 can be used with the present invention to obtain catheters with balloons coated with at least one therapeutically anti-infective agent against endocarditis.

Thus the present invention relates to catheter balloons for valvuloplasty balloon catheters which are coated with at least one therapeutically agent against infective endocarditis or with at least one anti-endocarditis agent.

More preferably the catheter balloon is only coated in its middle part which comes into contact with the heart valve(s). This saves costs and coating material and reduces exposure of the patient with additional pharmaceutically active agent which is present on parts of the catheter balloon which do not come into contact with tissue or heart valves. Thus a preferred embodiment of the present invention relates to valvuloplasty catheter balloons which are partly coated with an active agent as such, i.e. a pure active agent coating or with drug release system containing the active agent or with a combination of an active agent together with at least one carrier substance or matrix compound such as solubilizer, contrast agents, peptides, saccharides, vasodilators, esters, urea, shellac, acids such as salicylic acid, vitamins such as vitamin A, oligomeric or polymeric substances. The active agent is preferably an agent effective against endocarditis, especially against infective endocarditis.

Such balloons are provided with folds or wings forming mainly closed cavities if the balloon is in its deflated or compressed state. Upon expansion by inflation of the balloon the folds or wings bend outward and are capable of immediate release of substances contained within the folds or pressing said substances against the valves, respectively, upon contact with the valves.

Such balloons are advantageous because the at least one therapeutically anti-infective agent against endocarditis or the substances, respectively, enclosed within the folds are protected from untimely detachment during positioning of the catheter.

To protect the at least one therapeutically anti-infective agent against endocarditis from untimely detachment from the surface of the catheter balloon, the at least one therapeutically anti-infective agent may also be incorporated into or be embedded within a carrier substance, which preferably is a polymeric carrier. Shellac and other spongy or sponge-like carriers or polymers are most preferred. Such carriers and polymers are preferably able to absorb an active agent like a sponge and to release the absorbed active agent under pressure during dilatation.

For application of said carrier, shellac, or a further additional carrier or carriers to the surface of the catheter balloon, the carrier substance can be added to a solution of the at least one therapeutically anti-infective agent or can be applied as a second solution free of the at least one therapeutically anti-infective agent or once more with the at least one therapeutically anti-infective agent. Such solutions containing the at least one therapeutically anti-infective agent and/or shellac and optionally further carrier substances are then applied to the surface of the catheter balloon using conventional coating methods, in particular spattering, spraying, solvent welding or dipping methods as well as brush coating, vapour deposition, pipetting, electro-deposition and the like. Suitable additional carriers are such substances, which are used as balloon materials as well, in particular polymeric and polymerisable substances as listed below.

The at least one therapeutically anti-infective agent against endocarditis is embedded into shellac, preferably in the range of about 30% of the total amount being detached prematurely during insertion of the catheter and positioning of the catheter balloon resulting in a sufficiently high and therapeutically active amount of the at least one therapeutically active agents against endocarditis present on the balloon after the catheter balloon has been positioned correctly at its target position.

Thus, it is preferred to protect the at least one therapeutically anti-infective agent against endocarditis from untimely detachment by embedment into the shellac on the surface of the catheter balloon and, optionally, beneath the folds of the balloon.

Generally, an amount of 0.1 microgram to 30 micrograms of the at least one therapeutically anti-infective agent against endocarditis per square millimetre of the surface area of the catheter balloon to be coated can be applied to the surface of the balloon catheter, while an amount of 1 µg/mm² to 12 µg/mm² of the at least one therapeutically anti-infective agent against endocarditis is sufficient for achievement of the desired effect of prevention of endocarditis. Preferably, the amount of the at least one therapeutically anti-infective agent against endocarditis per square millimetre of balloon surface is between 2 µg/mm² and 12 µg/mm², more preferably between 3 µg/mm² and 9 µg/mm², still more preferably between 4 µg/mm² and 7 µg/mm², and most preferably between 5 µg/mm² and 6 µg/mm².

Preferably the total surface loading with the at least one therapeutically agent against infective endocarditis and optionally at least one carrier substance or matrix compound of the catheter balloon is between 1 µg/mm² and 50 µg/mm² and/or wherein the total surface loading with the at least one therapeutically agent against infective endocarditis of the catheter balloon is between 0.1 µg/mm² and 30 µg/mm².

In addition, a total amount of 10 to 1000 micrograms of the at least one therapeutically anti-infective agent against endocarditis per catheter balloon and most preferably 20 micrograms to 400 micrograms per catheter balloon is preferred depending on the selected active agent.

Preferably, the at least one therapeutically anti-infective agent against endocarditis has lipophilic properties. Therefore, solubility of said anti-infective agent against endocarditis in water is poor or very low, respectively, for example below 2.5 grams per litre at 25 °C in the case of cloramphenicol. The at least one therapeutically anti-infective agent against endocarditis has a distribution coefficient equal to or greater than 0.5 between octan-1-ol and water. Dissolution of the at least one therapeutically anti-infective agent against endocarditis is performed by the method according to the invention: The solution of the at least one therapeutically agent against infective endocarditis and, optionally, the solution of at least one carrier substance or the solution of the at least one therapeutically agent against infective endocarditis and optionally at least one carrier substance is prepared in acetone, ethyl acetate, ethanol, methanol, dimethyl sulfoxide, tetrahydrofuran, chloroform, methylene chloride and/or mixtures of the afore-mentioned solvents.

Dimethyl sulfoxide (DMSO), acetone, ethyl acetate, ethanol, methanol, tetrahydrofuran, chloroform, methylene chloride or mixtures thereof are used as solvents for the at least one therapeutically anti-infective agent against endocarditis. Materials used for the balloon catheter are such materials as listed further below, wherein the following polymers are particularly preferred: polyamides, block co-polymers of polyamide, polyether and polyester, polyurethanes, polyesters and polyolefins.

The inventive coating process can be performed in two alternative ways. A catheter balloon that preferably is an uncoated catheter balloon or a catheter balloon free of any releasable therapeutically anti-infective agents against endocarditis in its surface is provided. Next, a solution of the at least one therapeutically anti-infective agent against endocarditis together with shellac is prepared in a suitable solvent such as acetone, ethyl acetate, ethanol, methanol, dimethyl sulfoxide, tetrahydrofuran, chloroform, methylene chloride or the like and applied to the surface of the catheter balloon using conventional coating methods such as spray coating, dip coating etc. followed by a drying step in order to obtain a solid anti-infective agent-shellac coating on the surface of the catheter balloon (steps I, IIA, IIIA, and IV).

Alternatively, a first solution comprising the at least one therapeutically anti-infective agent against endocarditis plus a second solution of shellac, an oligomer or a polymer and most preferred a spongy or sponge-like polymer or oligomer are prepared, and both solutions are applied either concurrently or separately followed by a drying step in order to obtain a solid paclitaxel-shellac coating on the surface of the catheter balloon (steps I, IIB, IIIB, and IV). Sequential application of the solutions is divided by a drying step in-between.

Coating steps IIIA and IV or IIIB and IV, respectively, can be repeated several times in the inventive coating methods. Usually, the coating process is repeated once or twice or three times; nevertheless, said repetition is not compulsory. Even a single coating process can be sufficient for application of the required amounts of the at least one therapeutically anti-infective agent against endocarditis and, optionally, shellac to the catheter balloon.

Drying step IV can be conducted at room temperature or at elevated temperatures up to 50 °C. Drying step IV can be conducted at atmospheric or reduced pressure or at low vacuum to high vacuum. If coating step III (IIIA or IIIB) is repeated, drying step IV is conducted at room temperature and atmospheric pressure. Preferably, drying step IV is intensified, i.e. for a longer period of time such as up to 24 hours or in vacuum or at elevated temperature.

The catheter balloon is dilatable or expandable and is most preferably an angioplasty catheter balloon which could be used without crimped stent or with a crimpled stent. As stent, all kinds of common stents, such as self-expandable stents, not self-expandable stents, metal stents, polymer stents, biodegradable stents, bifurcation stents, uncoated (bare) stents, polymer coated stents, drug release coated stents, stents with a pure active agent coating etc. can be used.

The provided catheter balloon is normally a multi-fold catheter balloon deployed for valvuloplasty, which will also be coated under or within the folds. Moreover, selective coating or filling the folds is possible. Coating within or under the folds possesses the advantage that the coating and thus the at least one therapeutically anti-infective agent against endocarditis is protected against being washed off by the blood stream during insertion of the catheter and positioning of the balloon. Furthermore, the catheter balloon can be coated in its expanded sic inflated or deflated state.

Preferred solvents for the at least one therapeutically anti-infective agent against endocarditis and, optionally, shellac are volatile, easily removable solvents such as acetone, ethyl acetate, ethanol, methanol, dimethyl sulfoxide, tetrahydrofurane, chloroform and methylene chloride.

Total surface load with the at least one therapeutically anti-infective agent against endocarditis and, optionally, shellac of the catheter balloon is between 1 µg/mm² and 50 µg/mm² and/or wherein the total surface loading with the at least one therapeutically agent against infective endocarditis of the catheter balloon is between 0.1 µg/mm² and 30 µg/mm². Preferably, the amount of the at least one therapeutically anti-infective agent against endocarditis and, optionally, shellac present on the coated balloon surface is between 2 µg/mm² and 20 µg/mm² of balloon surface, more preferably between 3 µg/mm² and 18 µg/mm², still more preferably between 4 µg/mm² and 16 µg/mm², still more preferably between 5 µg/mm² and 14 µg/mm², and most preferably between 5.5 µg/mm² and 13 µg/mm² of balloon surface.

Before use of such a coated balloon catheter it can be sterilised optionally. Sterilisation is solved by the addition of a step V to the method according to the invention:
V) Sterilization of the catheter balloon that is coated with at least one therapeutically agent against endocarditis and optionally with at least one carrier substance.

### Sterilisation by treatment with ethylene oxide is preferred the most.

Moreover, the inventive coating method can, optionally, further comprise a step IB:
IB) Protecting the parts of the balloon catheter to be left uncoated with a removable protection sheet.

As the catheter balloon is only a part of the balloon catheter system, the surfaces of the balloon catheter to be left uncoated by the at least one therapeutically anti-infective agent against endocarditis and, optionally, shellac composite can be protected by a removable protection sheet such as a plastic bag or plastic foil. This results in only the catheter balloon being uncovered sic unprotected so that only the uncovered parts will be coated. After the coating process is completed, the protection sheet will be removed.

The inventive balloon coating method can, optionally, comprise a step VI:
VI) Protecting the coated catheter balloon with a removable protection cover.

The removable protection cover is useful for protection of the catheter balloon and, in particular, protection of the coating on the catheter balloon.

As described below in detail, the surface of the catheter balloon is textured, smooth, rough, harsh, provided with cavities or provided with channels open towards the outside of the balloon.

A preferred embodiment of the present invention relates to a valvuloplasty catheter, wherein the external surface of the catheter balloon is partially coated with the at least one therapeutically agent against infective endocarditis at the level of its middle segment. Moreover this middle section or the complete balloon surface is rough or textured. The term "middle segment" refers to this part of the surface of the catheter balloon which comes into contact or which can come into contact with tissue or a heart valve.

The coating solution containing the at least one therapeutically anti-infective agent against endocarditis can, optionally, contain at least one further carrier substance. Said at least one further carrier substance is selected from the group comprising: parylene C, parylene D, parylene N, parylene F, polyvalerolactones, poly-ε-decalactone, polylactonic acid, polyglycolic acid, polylactides, polyglycolides, copolymers of the polylactides and polyglycolides, poly_ε-caprolactone, polyhydroxybu-tyric acid, polyhydroxybutyrates, polyhydroxyvalerates, polyhydroxybutyrate-covalerate, poly(1,4-dioxane-2,3-dione), poly(1,3-dioxane-2-one), poly-para-dioxanone, polyanhydrides, polymaleic acid anhydride, polyhydroxymethacrylates, fibrin, polycyanoacrylate, polycaprolactone dimethylacrylates, poly-β-maleic acid, polycaprolactone butyl acrylates, multiblock polymers from oligocaprolactonedioles and oligodioxanonedioles, polyether ester multiblock polymers from PEG and poly(butylene terephthalate), polypivotolactones, polyglycolic acid trimethyl carbonates, polycaprolactone glycolides, poly(γ-ethyl glutamate), poly(DTH-iminocarbonate), poly(DTE-co-DT-carbonate), poly(bisphenol A-iminocarbonate), polyorthoesters, polyglycolic acid trimethyl-carbonate, polytrimethyl carbonates, polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide soft polyurethanes, polyurethanes having amino acid residues in the backbone, polyether esters, polyethylene oxide, polyalkene oxalates, polyorthoesters as well as their copolymers, lipids, carrageenans, fibrinogen, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and derivatives thereof, heparan sulfates and derivatives thereof, heparins, chondroitin sulfate, dextran, β-cyclodextrins, copolymers with PEG and polypropylene glycol, gum arabic, guar, gelatin, collagen N-hydroxysuccinimide, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylene, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, EPDM gums, fluorosilicones, carboxymethyl chitosans, polyaryletheretherketones, polyetheretherketones, polyethylene terephthalate, polyvalerates, shellac, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, epoxy resins, ABS resins, silicones, polysiloxanes, polydimethylsiloxanes, polyvinyl halogens and copolymers, cellulose ethers, cellulose triacetates, chitosans and copolymers and/or mixtures of the aforementioned polymers.

In case a textured surface of the catheter balloon is desired, the surface of the catheter balloon can be textured mechanically, chemically, electronically and/or by means of radiation to allow for an improved adhesion of the at least one therapeutically anti-infective agent against endocarditis and to allow for assistance in precipitation or crystallization of the at least one therapeutically anti-infective agent against endocarditis.

The surface of the catheter balloon can be modified within the range of nanometres to micrometres by texturing of the surface of the catheter balloon, i.e. provision of micro-rough surface structure. Surface texturing is preferably applied to the entire area of the catheter balloon to be coated. This can result in organized or random structures.

The catheter balloon may be composed of the following materials:
parylene C, parylene D, parylene N, parylene F, polyvalerolactones, poly-ε-decalactone, polylactonic acid, polyglycolic acid, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-covalerate, poly(1,4-dioxane-2,3-dione), poly(1,3-dioxane-2-one), poly-para-dioxanone, polyanhydrides, polymaleic acid anhydride, polyhydroxymethacrylates, fibrin, polycyanoacrylate, polycaprolactone dimethylacrylates, poly-β-maleic acid, polycaprolactone butyl acrylates, multiblock polymers from oligocaprolactonedioles and oligodioxanonedioles, polyether ester multiblock polymers from PEG and poly(butylene terephthalate), polypivotolactones, polyglycolic acid trimethyl carbonates, polycaprolactone glycolides, poly(γ-ethyl glutamate) poly(DTH-iminocarbonate), poly(DTE-co-DT-carbonate), poly(bisphenol A-iminocarbonate), polyorthoesters, polyglycolic acid trimethyl-carbonate, polytrimethyl carbonates, polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes having amino acid residues in the backbone, polyether ester, polyethylene oxide, polyalkene oxalates, polyorthoesters as well as their copolymers, lipids, carrageenans, fibrinogen, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and derivatives thereof, heparan sulfates and derivatives thereof, heparins, chondroitin sulfate, dextran, β-cyclodextrins, copolymers with PEG and polypropylene glycol, gum arabic, guar, gelatine, collagen N-hydroxysuccinimide, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylene, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, EPDM gums, fluorosilicones, carboxymethyl chitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, epoxy resins, ABS resins, silicones, polysiloxanes, polydimethylsiloxanes, polyvinyl halogens and copolymers, cellulose ethers, cellulose triacetates, chitosans and copolymers and/or mixtures of the aforementioned polymers.

Any kind of common coating process such as spray coating, brush coating, dip coating, vapour deposition, solvent welding, pipetting and the like can be used to apply the solution of the at least one therapeutically agent against endocarditis and, optionally, shellac or the solution of the at least one therapeutically anti-infective agent against endocarditis and the shellac solution to the balloon surface.

The content of the solution of the at least one therapeutically anti-infective agent against endocarditis is between 1 microgram to 1 milligram of the at least one therapeutically anti-infective agent against endocarditis per millilitre of solution, preferably between 10 µg to 1000 µg of the at least one therapeutically anti-infective agent per 1 ml solution, more preferably between 30 µg to 600 µg of the at least one therapeutically anti-infective agent per 1 ml solution, and most preferably between 50 µg to 200 µg of the at least one therapeutically anti-infective agent per 1 ml solution. For example, the solution of the at least one therapeutically anti-infective agent in ethanol, acetone, ethyl acetate or DMSO may be applied onto the balloon surface by means of spattering, dipping, plasma deposition, brushing, solvent welding or spraying. Although the entire surface of the catheter balloon is usually coated if a dipping method or plasma disposition are used, spattering, brushing and spraying may be used in case only a portion of the balloon surface is to be coated.

Furthermore, the present invention relates to expandable catheter balloons and in particular to multi-fold balloons for catheters coated according to the inventive method.

The catheter balloons are coated with a therapeutically anti-infective agent against endocarditis essentially as a pure substance. Thus, the inventive catheter balloons feature a layer consisting of an active agent in form of therapeutically anti-infective agent against endocarditis, optionally incorporated into a biopolymer, shellac, wherein only traces of solvent molecules are present in said layer, while optionally another active agent and/or another carrier substance may be present in a same or different amount as the at least one therapeutically anti-infective agent against endocarditis or shellac.

Due to the inventive coating method, the composite of the at least one therapeutically anti-infective agent against endocarditis dried on the surface of the catheter balloon, said composite can optionally contain shellac, is in a special state that is hard to characterize but seems to be indispensable for transfer to the valve tissue.

In the case of multi-fold balloons, a part of the at least one therapeutically anti-infective agent against endocarditis dried on the surface of the catheter balloon, said composite can optionally contain shellac, containing coating is provided underneath the folds when the balloon is in its compressed, i.e. deflated state. Said amount is sufficient to achieve the desired therapeutic success even if the remaining balloon surface despite having been uncovered is not coated with the at least on therapeutically anti-infective agent against endocarditis.

Thus, the present invention also relates to catheters comprising a catheter balloon coated according to the present invention with at least one therapeutically anti-infective agent against endocarditis and, optionally, shellac and, optionally, a further active agent and/or optionally a further carrier substance.

Such catheters are preferably used for treating stenotic heart valves, i.e. the aortic, mitral, pulmonary and tricuspid valves, by valvuloplasty and development of endocarditis due to bacterial and/or fungal infection as a direct result of the valvuloplasty procedure.

Coated balloon catheters according to the invention are deployed in balloon valvuloplasty of heart valves, i.e. mitral, aortic, pulmonary and tricuspid valve, and concurrent prevention of development of endocarditis by transfer of at least one therapeutically anti-infective agent against endocarditis to the valve tissue upon contact of the expanded balloon to the valve tissue. A physician guides the catheter through the femoral artery to the heart and into the narrowed aortic or mitral valve. In case of balloon valvuloplasty of the tricuspid or pulmonary valve, access will be though the femoral vein. Once in position, the balloon at the tip of the catheter is inflated. The balloon pushes open the aortic, mitral, pulmonary or tricuspid valve and stretches the valve opening, improving blood flow. The balloon is then deflated and the catheter with balloon is guided back out of the body. Balloon valvuloplasty relieves valve stenosis and its symptoms. In addition, the present invention prevents development of endocarditis to its coating with at least one therapeutically anti-infective agent against endocarditis and, optionally, a carrier substance.

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

### Examples

### Example 1

A commercially available valvuloplasty catheter with expandable balloon composed of a polyamide is provided. The surface of the catheter balloon is roughened in the range of nanometres to micrometres by means of sand blasting.

The antibiotic of the penicillin class, flucloxacillin (commercially available from Kin-bester Co., Ltd. or Hangzhou Dayangchem Co. Ltd., PR China) is dissolved in acetone at a concentration of 200 µg flucloxacillin and 100 µg shellac per ml of acetone. Optionally, flucloxacillin is dissolved together with shellac at a concentration of 100 µg shellac per millilitre of acetone. The solution of flucloxacillin and optionally shellac in acetone is sprayed onto the catheter balloon. Spraying is repeated three times after with drying of the coated balloon surface in-between each spraying step. Spraying is performed at room temperature and atmospheric pressure. After the final coating step, the catheter balloon is dried under reduced pressure and the complete valvuloplasty catheter system is sterilized with ethylene oxide. Then, the complete valvuloplasty catheter system including its coated balloon surface is covered with a protection cover and packed for shipping or storing.

### Example 2

A commercially available valvuloplasty catheter with expandable balloon composed of a polyamide is provided. The surface of the catheter balloon is roughened in the range of nanometres to micrometres by means of sand blasting.

Oxacillin (commercially available from e.g. Sigma-Aldrich Corp.) is provided at a concentration of 200 µg oxacillin dissolved in acetone. The surface of the balloon is coated with oxacillin according to the method described in Example 1 whereby flucloxacillin has been exchanged for oxacillin.

### Example 3

A commercially available valvuloplasty catheter with expandable balloon composed of a polyamide is provided. The surface of the catheter balloon is roughened in the range of nanometres to micrometres by means of sand blasting.

Vancomycin (commercially available from e.g. Sigma-Aldrich Corp.) is provided at a concentration of 200 µg vancomycin dissolved in acetone. The surface of the balloon is coated with vancomycin according to the method described in Example 1 whereby flucloxacillin has been exchanged for vancomycin.

### Example 4

A commercially available valvuloplasty catheter with expandable balloon composed of a polyamide is provided. The surface of the catheter balloon is roughened in the range of nanometres to micrometres by means of sand blasting.

Ceftriaxone (commercially available from e.g. TCI EUROPE N.V.) is provided at a concentration of 200 µg ceftriaxone dissolved in acetone. The surface of the balloon is coated with ceftriaxone according to the method described in Example 1 whereby flucloxacillin has been exchanged for ceftriaxone.

### Example 5

A commercially available valvuloplasty catheter with expandable balloon composed of a polyamide is provided. The surface of the catheter balloon is roughened in the range of nanometres to micrometres by means of sand blasting.

Amoxicillin (commercially available from e.g. Sigma-Aldrich Corp.) is provided at a concentration of 200 µg amoxicillin dissolved in acetone. The surface of the balloon is coated with amoxicillin according to the method described in Example 1 whereby flucloxacillin has been exchanged for amoxicillin.

### Example 6

A commercially available valvuloplasty catheter with expandable balloon composed of a polyamide is provided. The surface of the catheter balloon is roughened in the range of nanometres to micrometres by means of sand blasting.

Gentamicin (commercially available from e.g. Sigma-Aldrich Corp.) is provided at a concentration of 200 µg gentamicin dissolved in acetone. The surface of the balloon is coated with gentamicin according to the method described in Example 1 whereby flucloxacillin has been exchanged for gentamicin.

### Example 7

A commercially available valvuloplasty catheter with expandable balloon composed of a polyamide is provided. The surface of the catheter balloon is roughened in the range of nanometres to micrometres by means of sand blasting.

Netilmicin (commercially available from e.g. Sigma-Aldrich Corp.) is provided at a concentration of 200 µg netilmicin dissolved in acetone. The surface of the balloon is coated with netilmicin according to the method described in Example 1 whereby flucloxacillin has been exchanged for netilmicin.

### Example 8

A commercially available valvuloplasty catheter with expandable balloon composed of a polyamide is provided. The surface of the catheter balloon is roughened in the range of nanometres to micrometres by means of sand blasting.

Penicillin G (commercially available from e.g. Sigma-Aldrich Corp.) is provided at a concentration of 200 µg penicillin G dissolved in acetone. The surface of the balloon is coated with penicillin G according to the method described in Example 1 whereby flucloxacillin has been exchanged for penicillin G.

### Example 9

A commercially available valvuloplasty catheter with expandable balloon composed of a polyamide is provided. The surface of the catheter balloon is roughened in the range of nanometres to micrometres by means of sand blasting.

Rifampin (commercially available from e.g. Sigma-Aldrich Corp.) is provided at a concentration of 200 µg rifampin dissolved in acetone. The surface of the balloon is coated with rifampin according to the method described in Example 1 whereby flucloxacillin has been exchanged for rifampin.

### Example 10

A commercially available valvuloplasty catheter with expandable balloon composed of a polyamide is provided. The surface of the catheter balloon is roughened in the range of nanometres to micrometres by means of sand blasting.

Doxycycline (commercially available from e.g. Sigma-Aldrich Corp.) is provided at a concentration of 200 µg doxycycline dissolved in acetone. The surface of the balloon is coated with doxycycline according to the method described in Example 1 whereby flucloxacillin has been exchanged for doxycycline.

### Example 11

A commercially available valvuloplasty catheter with expandable balloon composed of a polyamide is provided. The surface of the catheter balloon is roughened in the range of nanometres to micrometres by means of sand blasting.

Erythromycin (commercially available from e.g. Sigma-Aldrich Corp.) is provided at a concentration of 200 µg erythromycin dissolved in acetone. The surface of the balloon is coated with Erythromycin according to the method described in Example 1 whereby flucloxacillin has been exchanged for erythromycin.

### Example 11

A commercially available valvuloplasty catheter with expandable balloon composed of a polyamide is provided. The surface of the catheter balloon is roughened in the range of nanometres to micrometres by means of sand blasting.

The balloon is expanded and, then, its surface is roughened by means of a so-called "chemical polishing" process, wherein a suspension of fine particles, preferably in the range of micrometres, is used in said process and said suspension is rubbed onto the surface of the expanded catheter balloon such that a roughened surface is created.

A solution of 300 µg of any therapeutically anti-infective agent from examples 1 to 10 in 1 ml of ethyl acetate and, optionally, a solution of 100 µg shellac in tertrahydrofu-rane (THF) is provided.

The roughened expanded catheter balloon is dipped several times into said solution of the anti-infective agent in ethyl acetate and dried at room temperature and atmospheric pressure after each dipping.

Next, optionally the shellac solution in THF is filled into a pipette and applied to the dry coating of the anti-infective agent on the balloon surface.

The total load of therapeutically anti-infective agent on the balloon surface is between 0.1 µg to 30 µg of anti-infective agent per mm² of coated balloon surface.

The balloon is provided with a protective sheath, sterilized and packaged under sterile conditions. The protective sheath is for protection of the anti-infective agent, which is coated on the surface of the expandable catheter balloon during storage and/or transport. Said sheath is removed by the physician prior to insertion of the valvuloplasty catheter into the blood vessel.

### Example 12

A commercially available valvuloplasty catheter with expandable balloon made of a polymer is provided. The catheter balloon consists of a block co-polymer of polyamide, polyether and polyester or of polyurethane, polyester or a polyolefin. The non-textured balloon surface is smooth and free of channels or cavities.

A solution of 210 µg of any therapeutically anti-infective agent from examples 1 to 10 and, optionally, 50 µg of shellac in 1.0 ml of ethanol, which has an ethanol content of at least 97.0 percent by volume, is prepared and applied to the horizontal area of the surface of the catheter balloon by brushing or spattering.

Next, the catheter balloon is thoroughly dried and the valvuloplasty catheter system sterilized with ethylene oxide. After sterilization, the balloon is provided with a protective sheath under sterile conditions. The protective sheath is intended for protection of the therapeutically anti-infective agent on the coated expandable catheter balloon during transport and storage. The sheath is removed prior to insertion of the catheter by the physician.

### Example 13

A coated catheter balloon for valvuloplasty is manufactured that has a coating of a therapeutically anti-infective agent of three micrograms per square millimetre of balloon surface.

The method described below is used for the coating of valvuloplasty balloon catheters for application in valve stenosis and prevention of development of endocarditis. The coating consists of a degradable, drug-eluting composite of any therapeutically anti-infective agent against endocarditis from examples 1 to 10 and, optionally, shellac with a surface loading of normally totalling 4 to 8 µg/mm² whereas, preferably, the mass portion of the therapeutically anti-infective agent against endocarditis from examples 1 to 10 is nominally 1 to 6 µg/mm². This coating layer is applied for release of an effectual portion of the therapeutically anti-infective agent from examples 1 to 10 to the valve tissue. The convenient effect of any therapeutically anti-effective agent from examples 1 to 10 is combined with fast release of the biodegradable carrier, shellac in the course of short-term release of any therapeutically anti-effective agent from examples 1 to 10 during balloon valvuloplasty.

### Process description

### 1. General handling

Quality assurance is conducted by coating and packaging being performed under sterile conditions in a controlled environment such as a laminar airflow cabinet.

### 2. Coating dilution

The coating dilution is a mixture of any therapeutically anti-infective agent from examples 1 to 10 and, optionally, shellac in a ratio of 1:1 in a necessary amount of ethyl alcohol. All raw materials undergo incoming inspection and are defined be specifications of raw materials.

### 3. Coating process

Before conducting the coating process the coating quantity must be calculated. The coating quantity is defined as the product of balloon surface and the specific load of 3 µg/mm². After unpacking of the catheter, the protection tube must be removed. The catheter is inserted into the working tube and adjusted in a controlled environment such as a laminar airflow cabinet. After removal of the protection cover, the catheter must be inserted to the coating device. Then, the catheter is fixed by a pneumatic actuator and visual inspection is conducted by use of a camera linked to a microscope. Afterwards, the required quantity of coating dilution is applied to the distribution rack by a pipette. The distribution rack is operated under the influence of a hot-air blower until the dilution will have been evenly distributed at the balloon surface. After evaporation of the solvent, ethyl alcohol, the coating is highly-adhesive to the surface. Post-processing is completed by drying via warm air and visual inspection of the surface by a camera linked to a microscope.

After completion of the coating process, the catheter must be removed from fixation by the pneumatic actuator; the balloon will be protected by a protection cover and inserted into the protection tube. Coated devices are stored in a controlled environment such as a laminar airflow cabinet until packaging.

The coating process for any therapeutically anti-infective agent from examples 1 to 10 and, optionally, shellac, is exclusively conducted using established and calibrated coating equipment plus certified raw materials.

The total load of any therapeutically anti-infective agent from examples 1 to 10 and, optionally, shellac, per square millimetre of balloon surface is 100 µg/mm², while the content of the therapeutically anti-infective agent from examples 1 to 10 is 30 µg/mm² per square millimetre of balloon surface.

### 4. Packaging process

The coated valvuloplasty catheters are packaged into sterile pouches suited for sterilisation by ethylene oxide in a controlled environment such as a laminar airflow cabinet. An indicator of ethylene oxide is added to every pouch to allow for control of successful sterilisation. Afterwards, pouches are labelled according to customer's order. All following packaging steps are conducted separately from the production area. Sterilisation by ethylene oxide is supported by a sterilisation validation.

### 5 Quality assurances

Raw materials for coating are ordered based on certified product specifications and will undergo incoming inspection. Incoming products will strictly be controlled regarding stains, surface defects and scratches by optical inspection. Before and after coating with any therapeutically anti-infective agent from examples 1 to 10 and, optionally, shellac, careful visual inspection is conducted by camera linked to a microscope. For determination of mass per balloon of the coating of any therapeutically anti-infective agent from examples 1 to 10 and, optionally, shellac, the difference in weight compared to an uncoated sample is determined. This will be done for every novel type of balloon size and/or fresh stock solution for coating. The loading of any therapeutically anti-infective agent from examples 1 to 10 and, optionally, shellac, per surface is validated.

### Example 14 Experimental model of calcific aortic stenosis

After approval by the Regional Animal Care Committee, male Watanabe rabbits weighing 2.5 to 3.0 kg were used in the experiments (Charles River Laboratories, Germany). Animals were assigned to a control (n=20) and a 0.25% cholesterol-fed group (n=20). All animals were fed *ad libitum* for 24 weeks. Control rabbits were fed a standard diet. Cholesterol-fed animals received a diet supplemented with 0.25% weight percentage of cholesterol (commercially available from e.g. Sigma-Aldrich Corp.).

One week before euthanasia, the rabbits received an intravenous injection of calcein to identify areas of new bone formation. After this 24-week period, the rabbits were anesthetized using intramuscular ketamine/xylazine (40/5 mg/kg) and then euthanized with intracardiac administration of 1 ml of Beuthanasia.

Immediately after dissection from the heart, one leaflet from each aortic valve and the aortic attachment was fixed in 4% buffered formalin for 24 hours and then embedded in paraffin. Paraffin embedded sections (6 pm) were cut and stained with hematoxy-lin and eosin, Masson trichrome, and elastin Van Gieson (EVG) stains for histopa-thologial examination.

The haematoxylin and eosin, Masson trichrome, and EVG stained hypercholesterolaemic aortic valves demonstrate a significant increase in leaflet thickness (0.70±0.2 mm, P<0.05). The aortic valve surface from control animals appeared normal, thin, and intact (0.06±0.04 mm).

### Example 15 Experimental model of endocarditis

Animal experiments were approved by the Regional Animal Care Committee. To establish endocarditis, anaesthesia was induced with a buprenorphine-ketamine-xylazine combination and maintained with isoflurane. A cut-down over the right carotid artery was performed, and a polyethylene catheter was positioned across the aortic valve of a 2.5- to 3.0-kg Watanabe rabbit. The catheter was secured in place for the duration of the experiment. Postoperative pain was managed with buprenorphine.

The rabbits were randomized to two groups: (i) an un-infected control group (n=20), which was euthanized at the initiation of infection to determine baseline bacterial burdens in aortic valve vegetations, (ii) an infected group that received 1 ml of 0.9% saline containing approximately 1.5 x 10⁷ colony forming units (CFU) of *Staphyloccocus aureus* strain COL intravenously forty-eight hours after positioning of the catheter.

Surviving rabbits were euthanized after 5 days. Aortic valve vegetations were removed. Tissues were homogenized in 0.5 ml of 0.9% saline, and 100-µl volumes were quantitatively cultured on blood agar to determine the number of bacteria present. The limit of detection of this method is 5 CFU per vegetation, or approximately 1.7 x log₁₀ CFU/g. Animals that died any time after infection were scored for mortality, but only those surviving beyond the first 48 h after infection were included in the analysis of tissue bacterial titres.

Mean organism titre (log₁₀ CFU/g±SD) for aortic valve vegetation was 8.7±0.9 for valves of animals of the infected group (n=20). Valves of animals of the control group had a mean organism titre below the detection limit of 1.7 x CFU/g. The difference between both groups was significant (P<0.05).

### Example 16 Experimental model of calcific aortic stenosis followed by endocarditis

Animal experiments were approved by the Regional Animal Care Committee. Animals of the control group (n=20) according to example 14 were subjected to balloon valvuloplasty of the aortic valve after 24 weeks by a standard protocol using valvuloplasty catheters with uncoated balloons. Animals were euthanized at the initiation of infection according to example 15. Cholesterol-fed animals (n=240) received a diet supplemented with 0.25% weight percentage of cholesterol (commercially available from e.g. Sigma-Aldrich Corp.) and were kept according to example 14. After 24 weeks, cholesterol-fed animals were subjected to balloon valvuloplasty of the aortic valve by a standard protocol followed by infection with *Staphyloccocus aureus* strain COL according to example 15. inventive Balloon catheters deployed for valvuloplasty of the aortic valve were coated as shown in Table 1:

**Table 1 Balloon valvuloplasty with therapeutically anti-infective agent coated to the balloon surface.**

| **Group number** | **Therapeutically anti-infective agent coated to the balloon surface** | **Coated according to** | **Number of animals** |
|---|---|---|---|
| 1 | Flucloxacillin | 1 | 20 |
| 2 | Oxacillin | 2 | 20 |
| 3 | Vancomycin | 3 | 20 |
| 4 | Ceftriaxone | 4 | 20 |
| 5 | Amoxicillin | 5 | 20 |
| 6 | Gentamicin | 6 | 20 |
| 7 | Netilmicin | 7 | 20 |
| 8 | Penicillin G | 8 | 20 |
| 9 | Rifampin | 9 | 20 |
| 10 | Doxycycline | 10 | 20 |
| 11 | Erythromycin | 11 | 20 |
| 12 | Uncoated | 12 | 20 |

After infection with *Staphyloccocus aureus* strain COL according to example 15 animals were kept according to example 15. Surviving rabbits were euthanized after 5 days; and aortic valve samples were prepared according to example 15. Mean organism titre (log₁₀ CFU/g±SD) of aortic valve vegetation was calculated according to example 15. Results are shown in Table 2.

**Table 2 Mean organism titre (log₁₀ CFU/g±SD) of aortic valve vegetation.**

| **Group number** | **Therapeutically anti-infective agent coated to the balloon surface** | **Mean organism titre (log₁₀ CFU/g±SD) of aortic valve vegetation** | **Number of animals** |
|---|---|---|---|
| 1 | Flucloxacillin | 3.6±1.4 | 19 |
| 2 | Oxacillin | 6.3±2.8 | 16 |
| 3 | Vancomycin | 5.9±1.7 | 19 |
| 4 | Ceftriaxone | 2.1±1.4 | 19 |
| 5 | Amoxicillin | 3.8±2.3 | 18 |
| 6 | Gentamicin | 4.9±3.2 | 18 |
| 7 | Netilmicin | 5.1±3.3 | 17 |
| 8 | Penicillin G | 3.2±2.7 | 18 |
| 9 | Rifampin | 4.3±1.5 | 17 |
| 10 | Doxycycline | 4.6±2.1 | 17 |
| 11 | Erythromycin | 3.4±2.9 | 18 |
| 12 | Uncoated | 9.5±1.3 | 14 |

## Claims

1. Method for coating a catheter balloon comprising the following steps:
I) providing an uncoated catheter balloon;
and
IIA) providing a solution of at least one therapeutically agent against endocarditis and optionally at least one carrier substance;
or
IIB) providing a solution of at least one therapeutically agent against endocarditis and optionally providing a solution of at least one carrier substance;
and
IIIA) coating the surface of the catheter balloon with the solution of at least one therapeutically agent against endocarditis and optionally at least one carrier substance;
or
IIIB) coating the surface of the catheter balloon with the solution of at least one therapeutically agent against endocarditis and subsequently with optionally a solution of at least one carrier substance or coating the surface of the catheter balloon with optionally a solution of at least one carrier substance and subsequently with the solution of at least one therapeutically agent against endocarditis;
IV) drying the coated catheter balloon.

2. Method according to claim 1, wherein the at least one therapeutically agent against infective endocarditis is selected from the group comprising or consisting of:
Ampicillin, bacampicillin, carbenicillin, indanyl, mezlocillin, piperacillin, ticarcillin, amoxicillin-clavulanic acid, ampicillin-sulbactam, benzylpenicillin, cloxacillin, dicloxacillin, methicillin, oxacillin, penicillin G, penicillin V, piperacillin, tazobactam, ticarcillin, +clavulanic acid, nafcillin, cephalosporin of the first generation, cefadroxil, cefazolin, cephalexin, cephalothin, cephapirin, cephradine, cefaclor, cefamandol, cefonicid, cefotetan, cefoxitin, cefprozil, ceftmetazole, cefuroxime, loracarbef, cefdinir, ceftibuten, cefoperazone, cefixime, cefotaxime, cefpodoxime proxetil, ceftazidime, ceftizoxime, ceftriaxone, cefepime, azithromycin, clarithromycin, clindamycin, dirithromycin, erythromycin, roxithromycin, telithromycin, cethromycin, spiramycin, ansymacin, oelandomycin, lincomycin, troleandomycin, cinoxacin, ciprofloxacin, enoxacin, gatifloxacin, grepafloxacin, levofloxacin, lomefloxacin, moxifloxacin, nalidixic, acid, norfloxacin, ofloxacin, sparfloxacin, trovafloxacin, oxolinic, acid, gemifloxacin, perfloxacin, imipenem-cilastatin, meropenem, aztreonam, amikacin, gentamicin, kanamycin, neomycin, netilmicin, streptomycin, tobramycin, paromomycin, teicoplanin, vancomycin, demeclocycline, doxycycline, methacycline, minocycline, oxytetracycline, tetracycline, chlortetracycline, tigecycline, mafenide, silver sulfadiazine, sulfacetamide, sulfadiazine, sulfamethoxazole, sulfasalazine, sulfisoxazole, trimethoprim-sulfamethoxazole, sulfamethizole, rifabutin, rifampin, rifapentine, linezolid, streptogramins, quinopristin, dalfopristin, bacitracin, chloramphenicol, fosfomycin, isoniazid, methenamine, metronidazol, mupirocin, nitrofurantoin, nitrofurazone, novobiocin, polymyxin, spectinomycin, trimethoprim, colistin, cycloserine, capreomycin, ethionamide, pyrazinamide, para-aminosalicyclic acid, erythromycin-2-acetate, erythromycin-2-stearate, erythromycin estolate, erythromycin ethylsuccinate, erythromycin glutamate, erythromycin lactic propionate, rifampicin, miconazole, ketoconazole, clotrimazole, econazole, bifonazole, butoconazole, fenticonazole, isoconazole, oxiconazole, sertaconazole, sulconazole, tioconazole, fluconazole, itroconauzole, isavuconazole, ravuconazole, posaconazole, voriconazole, teraconazole, abfungin, terbinafine, amorolfine, naftifine, butenafine, anidulafungin, caspofungin, micafungin, benzoic acid, ciclopirox olamine, tolnaftate, undecyclenic acid, 5-fluorocytosine, griseofulvin, haloprogin, fusidic acid, gramicidin, pristinamycin, ramoplanin, tyrotricin, natamycin, rimocidin, filipin, nystatin, amphotericin B, candicin, hamycin, sirolimus (rapamycin), biolimus A9, everolimus, myolimus, novolimus, pimecrolimus, ridaforolimus, tacrolimus FK 506, temsirolimus, zotarolimus, silver, copper and/or mixtures of the aforementioned agents.

3. Method according to claim 1, wherein the at least one carrier substance is selected from the group comprising:
parylene C, parylene D, parylene N, parylene F, polyvalerolactones, poly-ε-decalactone, polylactonic acid, polyglycolic acid, polylactides, polyglycolides, copolymers of the polylactides and polyglycolides, poly-ε-caprolactone, polyhydroxybutyric acid, polyhydroxybutyrates, polyhydroxyvalerates, polyhydroxybutyrate-co-valerate, poly(1,4-dioxane-2,3-dione), poly(1,3-dioxane-2-one), poly-para-dioxanone, polyanhydrides, polymaleic acid anhydride, polyhydroxymethacrylates, fibrin, polycyanoacrylate, polycaprolactone dimethylacrylates, poly-β-maleic acid, polycaprolactone butyl acrylates, multiblock polymers from oligocaprolactonedioles and oligodioxanonedioles, polyether ester multiblock polymers from PEG and poly(butylene terephthalate), polypivotolactones, polyglycolic acid trimethyl carbonates, polycaprolactone glycolides, poly(γ-ethyl glutamate), poly(DTH-iminocarbonate), poly(DTE-co-DTcarbonate), poly(bisphenol A-iminocarbonate), polyorthoesters, polyglycolic acid trimethyl-carbonate, polytrimethyl carbonates, polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide soft polyurethanes, polyurethanes having amino acid residues in the backbone, polyether esters, polyethylene oxide, polyalkene oxalates, polyorthoesters as well as their copolymers, lipids, carrageenans, fibrinogen, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and derivatives thereof, heparan sulfates and derivatives thereof, heparins, chondroitin sulfate, dextran, β-cyclodextrins, copolymers with PEG and polypropylene glycol, gum arabic, guar, gelatin, collagen N-hydroxysuccinimide, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylene, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, EPDM gums, fluorosilicones, carboxymethyl chitosans, polyary-letheretherketones, polyetheretherketones, polyethylene terephthalate, polyvalerates, shellac, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, epoxy resins, ABS resins, silicones, polysiloxanes, polydimethylsiloxanes, polyvinyl halogens and copolymers, cellulose ethers, cellulose triacetates, chitosans and copolymers and/or mixtures of the aforementioned polymers.

4. Method according to any one of the preceding claims, wherein the solution containing the at least one therapeutically agent against infective endocarditis has a content of the at least one therapeutically agent against infective endocarditis between 10 to 950 micrograms of therapeutically agent per one millilitre of solution.

5. Method according to any one of the preceding claims, wherein only the middle part of the catheter balloon is coated with the therapeutically agent.

6. Coated catheter balloon obtainable by the method according to any one of claims 1 to 5.

7. Valvuloplasty catheter comprising the coated catheter balloon, wherein the catheter balloon is coated with at least one therapeutically agent against infective endocarditis.

8. Valvuloplasty catheter according to claim 7, wherein the external surface of said catheter balloon is partially coated with the at least one therapeutically agent against infective endocarditis at the level of its middle segment.

9. Valvuloplasty catheter according to claim 7 or 8, wherein said catheter balloon takes the shape of an hour-glass shape upon inflation with its waist having a smaller diameter than that of its ends and only the surfaces of the middle segment of said catheter balloon that is the level of the hour-glass waist are coated with at least one therapeutically agent against infective endocarditis.

10. Valvuloplasty catheter according to claim 9, wherein said catheter balloon comprises a waist that has a crease shape and only the surfaces of the middle segment of said catheter balloon that is the level of the hour-glass waist are coated with at least one therapeutically agent against infective endocarditis.

11. Valvuloplasty catheter according to claim 9, wherein said catheter balloon comprises a waist that has a concave shape and only the surfaces of the middle segment of said catheter balloon that is the level of the hour-glass waist are coated with at least one therapeutically agent against infective endocarditis.

12. Valvuloplasty catheter according to any one of claims 7 - 11, wherein said catheter balloon comprises a distal end surface that has a shape that is the relief of the upper surfaces of heart valve leaflets and said distal end surface is coated with at least one therapeutically agent against infective endocarditis so if that said catheter balloon is inflated in the aortic root above the aortic valve and then gently pushed against its upper surfaces said catheter balloon delivers the drug coating to heart valves.

13. Valvuloplasty catheter according to claim 12, in which said catheter balloon comprises a distal end surface that comes in contact with the upper surfaces of the heart valves and said balloon comprises tiny microholes and/or pores said microholes and/or pores allowing the exit of the inflating solution when said balloon is expanded so that the inflation solution containing the at least one therapeutically agent against infective endocarditis soaks the upper surfaces of the valve leaflets.

14. Valvuloplasty catheter according to claim 10, wherein the catheter balloon comprises a waist that is so short and so deep that upon inflation it forms a so-called envelope in which the heart valve leaflets are captured and the surfaces of said envelope are coated with at least one therapeutically agent against infective endocarditis that is delivered this way to both upper and lower surfaces of the heart valves, which are inserted within.

15. Valvuloplasty catheter according to claim 14, wherein the catheter balloon comprises surfaces that form the waist of said catheter balloon and have in addition microscopic holes and/or pores with these microholes and/or pores allowing the exit of the inflating solution when said catheter balloon is expanded and said inflation solution contains the at least one therapeutically agent against infective endocarditis that soaks both the upper and lower surfaces of the heart valve leaflets.

16. Valvuloplasty catheter according to claim 14, wherein the catheter balloon comprises a waist that reaches the central lumen of the system and said waist separates completely the two spaces on its sides and said two spaces do not communicate and said spaces are inflated by two separate lumens and in which the proximal balloon is inflated first and the distal surface of the proximal balloon, with said distal surface coated with at least one therapeutically agent against infective endocarditis, is pushed against and contacts the upper surfaces of the heart valve leaflets; immediately afterwards the distal balloon is inflated forming the envelope in which the leaflets are captured; the proximal surface of the distal balloon is coated with at least one therapeutically agent and comes in contact with the lower surfaces of the heart valve leaflets delivering the at least one therapeutically agent.

17. Valvuloplasty catheter according to claim 14, wherein the catheter balloon comprises in addition a hollow centre along the long axis of said balloon so that upon inflation of said balloon it is expanded and creates a central cylindrical lumen; in the internal surface of said lumen and at the level of the waist of the balloon there are 2 or 3 artificial crests that have the shape of the aortic leaflets; when the balloon is deflated and shrunk these crests are contained within the also shrunk central lumen of the balloon, but as the balloon is inflated the walls of the central cylindrical lumen are apposed and the lumen appears, the 'artificial' leaflets drop within it and function as a temporary 'artificial' aortic valve; as the hour-glass shaped balloon is inflated the aortic valve leaflets are captured within the envelope that is formed in the waist and coating with at least one therapeutically agent against infective endocarditis from the attendant balloon surfaces is delivered to the leaflet tissues; in addition, with the inflation and expansion of the balloon the central cylindrical lumen running its long axis from end to end opens allowing blood flow with each heart beat from the left ventricle to the aorta through the temporary 'artificial' aortic valve that drops and functions within the lumen; backflow of the blood to the left ventricle is not possible through this 'artificial' valve.

18. Use of the valvuloplasty catheter formed and produced according to claims 7 to 17 to prepare a means for the treatment of stenosis of heart valves and prevention of infective endocarditis.
